# EUROPEAN PATENT SPECIFICATION

(11) **EP 3 527 152 B1**
(45) Date of publication and mention of the grant of the patent: **23.12.2020**
(21) Application number: 18157011.0
(22) Date of filing: 15.02.2018
(51) Int. Cl.: A61B 17/3201, A61B 17/29, A61B 17/295, A61B 17/24, A61B 17/00, A61B 17/32

(54) **TOOL FOR A MICROINVASIVE SURGICAL INSTRUMENT AND MICROINVASIVE SURGICAL INSTRUMENT**
WERKZEUG FÜR EIN MIKROINVASIVES CHIRURGISCHES INSTRUMENT UND MIKROINVASIVES CHIRURGISCHES INSTRUMENT
OUTIL POUR UN INSTRUMENT CHIRURGICAL MICROINVASIF ET INSTRUMENT CHIRURGICAL MICROINVASIF

(43) Date of publication of application: 21.08.2019
(73) Proprietor: University of Dundee, Dundee DD1 4HN (GB)
(72) Inventor: Coleman, Stuart, Dundee, DD2 1FD (GB); Ross, Pete, Dundee, DD1 9SY (GB)
(74) Representative: Wimmer, Stephan

(56) References cited:
- WO-A1-95/15124
- CN-A- 103 479 413
- US-A- 2 127 190
- US-A1- 2013 006 242

## Description

The present invention refers to a tool for a microinvasive surgical instrument and to a microinvasive surgical instrument comprising such tool.

In a typical endoscopic and other microinvasive surgical procedure, a number of different actions or measures are performed consecutively, comprising elevation, grasping or gripping, cutting etc. For these different actions or measures, different tools are used. When changing from a first tool to a second tool, the surgeon typically withdraws a first instrument comprising the first tool and inserts a second instrument comprising the second tool. Each and every change of instrument can cause trauma, consumes time and increases the period of anesthetization for the patient.

Therefore, a number of attempts have been made to develop multifunctional tools and instruments which can carry out multiple tasks and allow performance of a number of different actions or measures without the need of exchange of instrument.

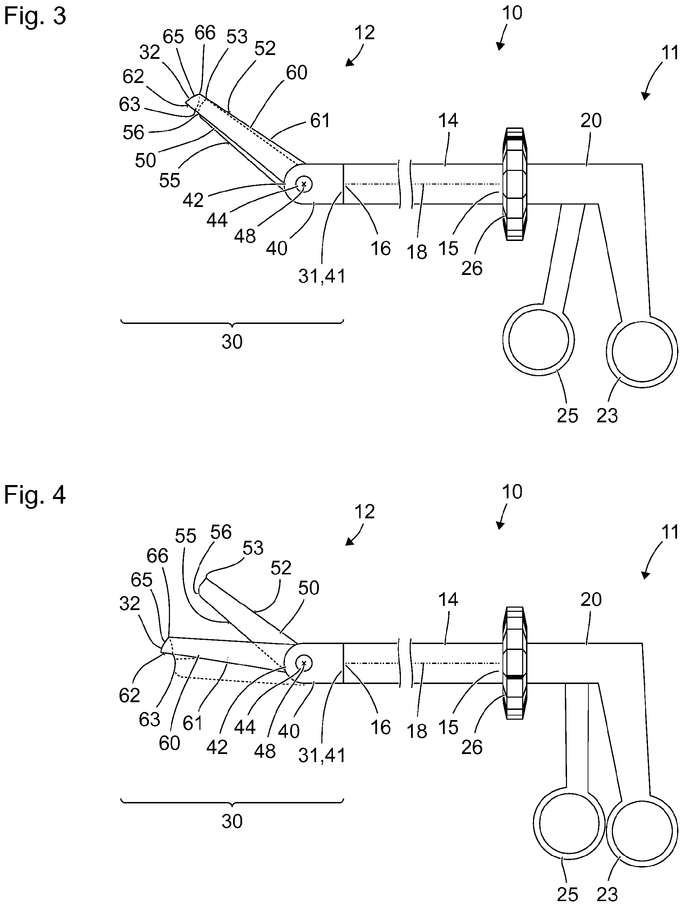

In US 5,456,684, a multifunctional minimally invasive surgical instrument is described. The instrument comprises jaws and a **blade slidable** between a retracted position within a support tube and an extended cutting position on jaw (column 3, lines 60 through 66, figures 3, 9, 12).

In US 5,984,939, a multifunctional grasping instrument with a cutting member is described. The multifunctional grasping instruments comprises two jaws with laterally opposed inner surfaces at a **grasping portion** and blades proximally spaced from the grasping surfaces (column 5, lines 48 through 60; figure 4).

In US 2007/0179525 A1, a multifunctional tool is described. The multifunctional tool comprises two jaws (paragraph [0024]). The jaws include surfaces that can effectuate grasping or dissecting or surfaces that can effectuate cutting by scissors action. Both jaws are pivotable about a **pivot axis**, thereby generating a **clamping** movement (paragraph [0177], figures 17b, 19b). Both jaws are pivotable about **pivot axes** orthogonal to the pivot axis thereby accomplishing **scissors** action (paragraphs [0179], [0186], figures 17a, 17c).

In WO 2007/120063 A2 (also published as EP 2 160 139 B1), a multifunctional surgical instrument is described. The multifunctional surgical instrument comprises **replaceable working parts** (page 3, lines 10 through 13). Depending on the shape of the working parts, the multifunctional instrument can be used for different purposes (page 3, lines 24 through 30).

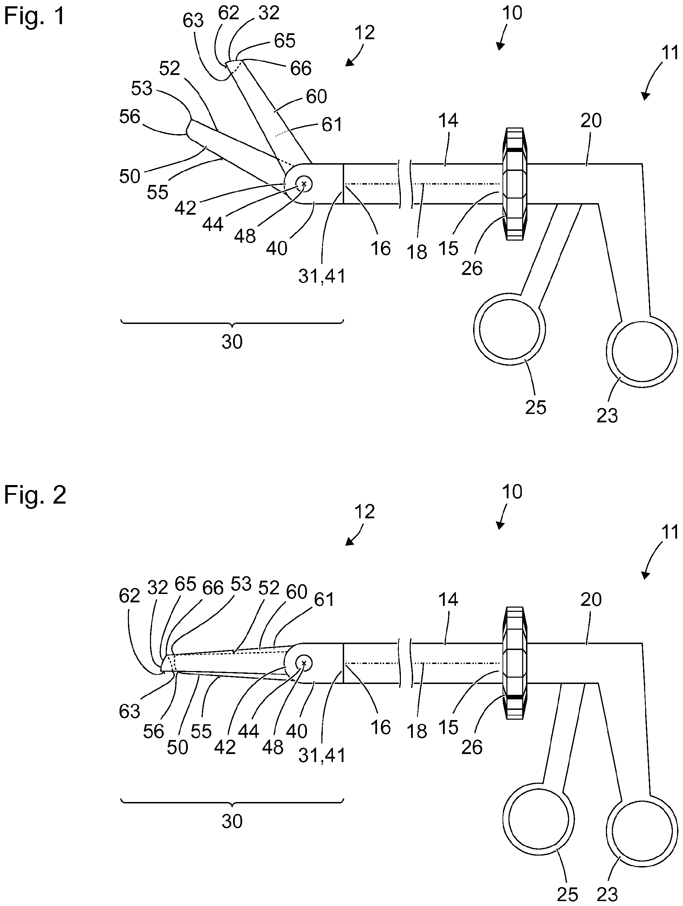

In WO 2014/049543 A2, a multifunctional surgical device is described. The multifunctional surgical device comprises at its distal end a multifunctional tool with a multifaceted jaw (paragraph [036] and figures 1a through 1e). The multifaceted **jaw** can be **rotationally** indexed along its longer axis to achieve 2, 3, 4 or more positions. Each indexation or orientation of the jaw couples one of its facet profiles with the complementary surface of a pivotable jaw, thus allowing for a different surgical function chosen among grasping, scissoring, dissecting, needle driving.

WO 95/15124 A1 and CN 103479413 A disclose tools with a jaw providing the shape of a 'U' or 'O' at least partially encircling a fenestration.

It is an object of the present invention to provide an improved tool for a microinvasive surgical instrument and a microinvasive surgical instrument, in particular accomplishing multiple surgical tasks.

This object is solved by the subject matter of the independent claim.

Further embodiments are described in the dependent claims.

A tool for a microinvasive surgical instrument comprises a body, the proximal end of the body member being mechanically connected to or being configured to be mechanically coupled to a distal end of a shaft of a microinvasive surgical instrument; a first jaw mechanically connected to the body member in an articulated manner; a second jaw mechanically connected to the body member in an articulated manner, wherein the first jaw and the second jaw are configured to be pivoted independently from each other, wherein the second jaw provides the shape of a 'U' or 'O' at least partially encircling a fenestration, wherein the tool provides open configurations with an angular distance between the first jaw and the second jaw, and wherein the tool provides a closed configuration with the first jaw at least partially accommodated in or capping the fenestration of the second jaw.

In particular, the tool is provided and configured for a microinvasive surgical instrument for applications in otorhinolaryngology. The proximal end of the body member of the tool can be configured for a detachable mechanical coupling to the distal end of the shaft of the microinvasive surgical instrument, for instance by means of a bayonet joint or by two corresponding threads at the distal end of the shaft and at the proximal end of the body member. As an alternative, a permanent mechanical connection between the proximal end of the body member and the distal end of the shaft can be provided. In particular, the body member can be soldered or welded or glued to the distal end of the shaft or the body member and the shaft can be monolithic.

One or two hinges connect the first jaw and the second jaw to the body member. In particular, the first jaw is pivotable about a first axis, and the second jaw is pivotable about a second axis, wherein the first axis and the second axis are parallel or essentially parallel or identical. In particular, the first axis and the second axis are orthogonal to the longitudinal axis of a shaft mechanically connected or coupled to the body member.

In particular, the tool does not provide any direct mechanical coupling between the first jaw and the second jaw that would directly couple both jaws angular motions about their respective pivot axes. However, the tool can be part of a microinvasive surgical instrument or can be integrated into a microinvasive surgical instrument which couples the first jaw's and the second jaw's angular motions about their respective pivot axes. For instance, the tool can be coupled to a handle assembly (via the shaft mentioned above and transmission members within the shaft) which handle assembly mechanically couples both jaws' angular motions about their pivot axes to each other. For example, the handle assembly can provide a first manually moveable handle or control controlling the angular distance between the jaws and a second manually moveable handle or control controlling the orientation of both jaws or of a median orientation of the jaws. In this case manipulation of the first movable handle or control can cause angular motions of both jaws in opposite directions or of a single jaw, and manipulation of the second handle or control can cause angular motions of both jaws in the same direction.

If the second jaw provides the shape of a 'U', the open ends of the 'U' can be oriented towards the pivot axis of the second jaw. In this case, the second jaw together with its pivot axis or, to be more precise, with the axle defining the second jaw's pivot axis encircles the fenestration. In this case, the first jaw pivoting about the same pivot axis can be configured to be completely passed through the fenestration.

As an alternative, the second jaw can provide the shape of an 'O' entirely encircling the fenestration.

In particular, the tool provides a continuous range of open configurations with a continuous range of angular distances between the first and second jaws. In the open configurations of the tool, tissue or another object can be positioned between the first and second jaws and can be grasped or cut by moving the jaws towards each other. When tissue or another object is positioned between the first and second jaws of the tool in an open configuration, and the tool is transformed to its closed configuration by approaching the first and second jaws to each other, the jaws can be configured to cut or punch out a piece of tissue.

In particular, the shape of the second jaw corresponds to the shape of a lateral edge of the first jaw.

The shape of the second jaw corresponds or essentially corresponds to the shape of the lateral edge of the first jaw when there is at least one configuration in which the second jaw essentially or entirely abuts on the lateral edge of the first jaw or in which the distance between the lateral edge of the first jaw and the surface of the second jaw is less than a predetermined small value of 100 micrometer or 50 micrometer or 20 micrometer or 10 micrometer or less.

In particular, the shape of the second jaw corresponds to or essentially corresponds to the shape of the edge of the first jaw, when in at least one configuration of the tool the first jaw fills or essentially fills the entire fenestration in the second jaw.

In particular, each of the first jaw and the second jaw is monolithic.

A jaw is monolithic, if it is originally made from a single part or workpiece. As an alternative, a jaw is also monolithic, if it is made from a plurality of parts or workpieces which are rigidly and permanently connected to each other, for instance welded, soldered or glued.

In particular, each of the first jaw and the second jaw comprises merely one member and this member of each jaw is rigid.

In particular, each of the first jaw and the second jaw provides exactly one degree of freedom relative to the body member.

In particular, the single degree of freedom of each first and second jaw is the rotation about the pivot axis already mentioned above.

In particular, one or more axles, shafts or pins guided in one or more holes connect the first jaw and the second jaw to the body member in an articulate manner.

In particular, in the closed configuration of a tool, the second jaw can essentially abut on a U-shaped or O-shaped region extending along a lateral edge of the first jaw.

In particular, the first jaw provides, at sides averted from each other, a first surface area and a second surface area, the second jaw provides two elongate surface areas at parallel or essentially parallel regions of the second jaw, and the tool provides configurations in which edges of each of the elongate surface areas at the second jaw face corresponding edges of the first surface area at the first jaw.

The two elongate surface areas can be part of one contiguous U-shaped or O-shaped surface area. In this case, the edges of each of the elongate surface areas at the second jaw are part of a U-shaped or O-shaped edge extending at the inner side of the second jaw and encircling the fenestration in the second jaw.

In particular, the first surface area at the first jaw and the two elongate surface areas or the contiguous U-shaped or O-shaped surface area at the second jaw are grasping surface areas provided to exert moderate pressure on a piece of tissue grasped by the tool. Friction between the grasping surface areas and tissue can be increased by corrugation of the grasping surface areas.

Edges of the grasping surface areas can be blunt or cutting. In the latter case, it depends on the force or pressure exerted on tissue whether the tissue is merely grasped or cut by the tool.

In a tool as it is described herein, the second jaw provides, in particular, a cutting edge exposed and averted from the first jaw in at least one configuration of the tool.

In particular, the cutting edge at the second jaw is exposed and averted from the first jaw and, therefore, ready to cut or scrape tissue like a curette in all configurations of the tool or in a predetermined range of configurations of the tool. The configuration is, in particular, a open or semi-open configuration of the tool. When used as a curette, the first jaw may be used to conveniently eject any accumulated tissue from the second jaw by closing the jaws together.

In particular, each of the first and second jaws is essentially wedge-shaped.

In particular, the height of each of the first and second jaws decreases from its pivot axis to the distal end of the tool.

The height of a jaw is measured in a direction orthogonal to the pivot axis of the jaw and orthogonal to the longitudinal direction of the jaw.

In a tool as it is described herein in the closed configuration of the tool, the contour of the first and second jaws is, in particular, essentially wedge-shaped.

Essentially wedge-shaped jaws and, in particular, a wedge-shape of both jaws together in their closed configuration facilitates the utilization of the tool for elevation or blunt dissection of tissue. Furthermore, because the first jaws fits inside of the second jaw when closed, the total height of the closed jaws may be kept very small. When the jaws are closed together, any sharp cutting edges are shielded from contact with tissue. Thus, the tool can be utilized both for blunt dissection and for grasping and/or cutting tissue.

In a tool as it is described herein, the cross section of the fenestration at least partially encircled by the second jaw is, in particular, complementary to the cross section of the first jaw such that the first jaw can be passed through the fenestration from a first side of the second jaw to a second side of the second jaw averted from the first side of the second jaw.

When the first jaw can be passed through the fenestration in the second jaw, the tool can provide two completely different open configurations (viz. configurations with angular distance between the jaws), wherein the first jaw is situated at two opposite sides of the second jaw.

In a tool as it is described herein, in particular, the first jaw provides, at sides averted from each other, a first surface area with a first U-shaped or O-shaped edge and a second surface area with a second U-shaped or O-shaped edge; the second jaw provides, at sides averted from each other, a first U-shaped or O-shaped surface area with a first U-shaped or O-shaped edge and a second U-shaped or O-shaped surface area with a second U-shaped or O-shaped edge; in a first configuration of the tool, the first edge at the second jaw faces the first edge at the first jaw, and in a second configuration of the tool, the second edge at the second jaw faces second edge at the first jaw.

In particular, the tool is transformed from the first configuration to the second configuration by passing the first jaw through the fenestration at least partially encircled by the second jaw.

In a tool as it is described herein, in particular, the first jaw provides, at sides averted from each other, a first surface area with a U-shaped or O-shaped cutting edge and a second surface area with a U-shaped or O-shaped blunt edge; the second jaw provides, at sides averted from each other, a first U-shaped or O-shaped surface area with a U-shaped or O-shaped cutting edge and a second U-shaped or O-shaped surface area with a U-shaped or O-shaped blunt edge; in a cutting open configuration of the tool, the cutting edge at the second jaw faces the cutting edge at the first jaw; and in a grasping open configuration of the tool, the blunt edge at the second jaw faces the blunt edge at the first jaw.

Both the first and second surface areas at both the first jaw and the second jaw can be smooth (in particular planar or essentially) or corrugated, wherein corrugated surface areas can reduce the risk that tissue intended to be grasped or cut by the tool slips away when the jaws approach each other.

The tool can be transformed from the cutting open configuration to the grasping open configuration by passing the first jaw through the fenestration at least partially encircled by the second jaw.

In particular, in a cutting action transforming a tool as it is described herein from the cutting open configuration towards the closed configuration, the cutting edge at the first jaw slides against or passes the cutting edge at the second jaw in a tissue cutting manner.

In particular, in a grasping action transforming a tool as it is described herein from the grasping open configuration to the closed configuration, the blunt edge at the first jaw is moved towards the blunt edge at the second jaw.

In a tool as it is described herein, in particular, both the first jaw and the second jaw are pivotable about the same pivot axis.

In particular, both the tool's grasping action mentioned above and the tool's cutting action mentioned above are conducted by pivoting the first jaw relative to the second jaw and/or the second jaw relative to the first jaw about the same axes or about the same axis.

A tool as it is described herein provides, in particular, at least two of configurations of an elevating closed configuration in which the first jaw is arranged at least partially inside the fenestration in the second jaw, wherein the second jaw, by abutting on or being located close to a cutting edge at the first jaw, prevents the cutting edge at the first jaw from cutting tissue, and the first jaw, by abutting on or being located close to a cutting edge at the second jaw, prevents the cutting edge at the second jaw from cutting tissue; a grasping open configuration, tissue being grasped between the first jaw and the second jaw when the first and second jaws are moved towards each other from the grasping open configuration towards the closed configuration; a cutting open configuration, tissue being cut between cutting edges at the first and second jaws sliding against or passing each other when the first and second jaws are moved towards each other from the cutting open configuration towards the closed configuration; a curettage configuration in which a cutting edge at the second jaw averted from the first jaw is exposed and configured to cut tissue when the tool is moved parallel to a surface of the tissue.

The tool providing two or more different functional configurations is able to replace a corresponding number of different tools or medical instruments. Thus, the tool can reduce the number of exchanges or replacements of microinvasive instruments during surgery.

In a tool as it is described herein, in particular, in the elevating closed configuration, the first and second jaws are in any angular position within a first predetermined range of angular positions relative to the body member, with a first predetermined angular relation between the first jaw and the second jaw; in the grasping open configuration, each of the first jaw and the second jaw is in any angular position within a respective second predetermined range of angular positions relative to the body member, with a second predetermined angular relation between the first jaw and the second jaw; in the cutting open configuration, each of the first jaw and the second jaw is in any angular position within a respective third predetermined range of angular positions relative to the body member, with a third predetermined angular relation between the first jaw and the second jaw; in the curettage configuration, the first and second jaws are in any angular position within a fourth predetermined range of angular positions relative to the body member, with a fourth predetermined angular relation between the first jaw and the second jaw.

The different functional configurations of the tool require different angular relations between the first and second jaws. In each of the different functional configurations, the angular positions of both jaws relative to the body member (and, thus, to the shaft of a microinvasive surgical instrument comprising the tool), can be varied or set to arbitrary values the surgeon prefers or requires). Since both jaws are pivotable, the tool allows the surgeon to set optimum angles between the jaws and the body member or the shaft for almost every application.

A microinvasive surgical instrument comprises a shaft with a proximal end and a distal end; a tool as it is described herein, wherein the proximal end of the body member of the tool is mechanically connected to or is configured to be mechanically coupled to the distal end of the shaft; and a handle assembly comprising a fixed handle mechanically connected to or configured to be mechanically coupled to the proximal end of the shaft and controls manually movable relative to the fixed handle and mechanically coupled to the first and second jaws.

In particular, the microinvasive surgical instrument is provided and configured for applications in otorhinolaryngology. The shaft of the microinvasive surgical instrument can be flexible (elastically and/or plastically bendable) or rigid, straight or curved.

Each of the tool and the handle assembly can be permanently connected to or detachably coupled to the respective end of the shaft. Transmission means inside the shaft mechanically couple the controls of the handle assembly to the jaws of the tool.

In a microinvasive surgical instrument as it is described herein, the handle assembly is, in particular, configured to fix at least one of the first jaw and the second jaw in a manually adjustable position within a predetermined range of positions relative to the body member.

In particular, the handle assembly comprises two controls each of which is exclusively coupled to one of the first and second jaws of the tool. As an alternative, the handle assembly can comprise two controls both of which are coupled to both jaws of the tool, wherein a first control controls a direction or orientation or angular position of both jaws in the closed configuration of the tool and the second control controls a more or less symmetric motion of both jaws towards each other or away from each other. As another alternative, the handle assembly can comprise two controls wherein a first control is coupled to both jaws of the tool and controls a direction or orientation or angular position of both jaws, and a second control is coupled to one jaw so that it may be used to open or close that jaw relative to the other jaw. In all cases and in further cases not mentioned here, one control can be, for instance, a wheel manually rotatable about the longitudinal axis of the proximal end of the shaft or about another axis, and the other control can be, for instance, a handle pivotable relative to the fixed handle about an axis orthogonal to the longitudinal axis of the proximal end of the shaft. In any case, at least one control can be coupled to one jaw or to both jaws in a self-locking manner, for instance via a lead screw.

### Short description of the figures

Embodiments will be described below with reference to the enclosed figures.
- Figure 1: shows a schematic representation of a microinvasive surgical instrument;
- Figure 2: shows another schematic representation of the instrument displayed in figure 1;
- Figure 3: shows another schematic representation of the instrument displayed in figures 1 and 2;
- Figure 4: shows another schematic representation of the instrument displayed in figures 1 through 3;
- Figure 5: shows a schematic representation of a cross section of the tool of the instrument displayed in figures 1 through 4;
- Figure 6: shows another schematic representation of a cross section of the tool displayed in figure 5;
- Figure 7: shows another schematic representation of a cross section of the tool displayed in figures 5 and 6;
- Figure 8: shows another schematic representation of the tool displayed in figures 5 through 7;
- Figure 9: shows a schematic representation of another microinvasive surgical instrument.

### Description of embodiments

Figure 1 shows a schematic representation of a microinvasive surgical instrument 10 with a proximal end 11 and a distal end 12. The microinvasive surgical instrument 10 comprises a shaft 14 with a proximal end 15 and a distal end 16. In the example displayed in figure 1, the shaft 14 is straight and rigid and has a longitudinal axis 18 parallel to the plane of projection of figure 1.

The proximal end 15 of the shaft 14 is permanently mechanically connected or detachably coupled to a handle assembly 20 forming the proximal end 11 of the instrument 10. The handle assembly 20 comprises a fixed handle 23 which is rigidly and permanently connected or rigidly but detachably coupled to the proximal end 15 of the shaft 14. Furthermore, the handle assembly 20 comprises a moveable handle 25 which is pivotable relative to the fixed handle 23 about an axis orthogonal to the longitudinal axis 18 of the shaft 14 and orthogonal to the plane of projection of figure 1. Furthermore, the handle assembly 20 comprises a control 26. In the example displayed in figure 1, the control 26 is a wheel rotatable about the longitudinal axis 18 of the shaft 14 and located between the proximal end 15 of the shaft 14 and the handle assembly 20.

The distal end 16 of the shaft 14 is permanently mechanically connected or detachably coupled to a tool 30. To be more specific, a proximal end 31 of the tool 30 is permanently connected or detachably coupled to the distal end 16 of the shaft 14. A distal end 32 of the tool 30 forms the distal end 12 of the instrument 10.

The tool 30 comprises a body member 40. The proximal end 41 of the body member 40 forms the proximal end 31 of the tool 30. Close to the distal end 42 of the body member 40, an axle 44 is provided. The axle 44 defines an axis 48 orthogonal to the plane of projection of figure 1 and connects the body member 40 to a first jaw 50 and to a second jaw 60 in an articulated manner. In other words, the axle 44 forms a hinge between the distal end of the body member 40 and proximal ends of the first jaw 50 and the second jaw 60.

Both jaws 50, 60 can be moved (pivoted about the axis 48) independent from each other. The jaws 50, 60 are mechanically coupled to the moveable handle 25 and the control 26 of the handle assembly 20. For instance, the first jaw 50 is coupled to the moveable handle 25, and the second jaw 60 is coupled to the control 26 of the handle assembly 20. A surgeon can pivot the second jaw 60 by manually rotating the control 26 about the axis 18 of the shaft 14 and pivot the first jaw 50 by manually moving the moveable handle 25 relative to the fixed handle 23 of the handle assembly 20. As an alternative, the control 26 of the handle assembly 20 can be mechanically coupled to both the first jaw 50 and the second jaw 60 such that any motion of the control 26 of the handle assembly 20 causes both first and second jaws 50, 60 to pivot in the same direction, while the movable handle 25 causes the first jaw 50 to pivot without moving the second jaw 60. This alternative configuration allows the second jaw to be fixed in place while it is used for curettage.

The first jaw 50 provides, at two sides averted from each other, a first surface area 52 and a second surface area 55 averted from the first surface area 52. In the configuration displayed in figure 1, the first surface area 52 of the first jaw 50 is oriented towards, or faces the second jaw 60.

The second jaw 60 provides the shape of a 'U' the ends of which are mechanically connected to the body member 40 by means of the axle 44 in an articulated manner. The second jaw 60 (to be more specific: together with the axle 44) encircles a fenestration 61. Two arms of the U-shaped second jaw 60 are parallel or essentially parallel to the plane of projection of figure 1. A third section of the U-shaped second jaw 60 is orthogonal or essentially orthogonal to the plane of projection of figure 1, and the hidden part of the contour of its cross section is indicated by a dashed line in figure 1.

The second jaw 60 provides, at two sides averted from each other, a first U-shaped surface area 62 and a second U-shaped surface area 65. In the configuration displayed in figure 1, the first surface area 62 of the second jaw 60 is oriented towards, or faces the first jaw 50.

An edge 53 at the first jaw 50 forms a lateral boundary of the first surface area 52 of the first jaw 50. An edge 63 at the second jaw 60 forms a lateral boundary between the first surface 62 of the second jaw 60 and the fenestration 61 encircled by the first jaw 60. Both the edge 53 of the first surface area 52 at the first jaw 50 and the edge 53 at the second surface area 62 at the second jaw 60 are configured as cutting edges.

Another edge 56 at the first jaw 50 is blunt and forms a lateral boundary of the second surface area 55 of the first jaw 50. In the example displayed in figure 1, the second surface area 65 of the second jaw 60 provides another cutting edge 66. In the configuration displayed in figure 1, the cutting edge 66, together with the second surface area 65, is averted from the first jaw 50.

When the tool 30 - together with the entire instrument 10 - is moved parallel to a surface of tissue in a proximal direction (in figure 1: to the right), the cutting edge 66 at the second jaw can cut tissue like a conventional loop curette. If, while using the instrument like a loop curette, the fenestration 61 becomes filled with excised tissue, obscuring the users vision or the instrument's functionality, the first jaw 50 may be closed in order to eject the collected tissue from the fenestration 61.

In the example displayed in figure 1, the cross section (in a plane comprising the axis 48 defined by the axle 44) of the fenestration 61 in the second jaw 60 corresponds to the cross section of the first jaw 50 such that the first jaw 50 can be passed through the fenestration 61 in the second jaw 60. Clearance between the first jaw 50 and the second jaw 60 is such that tissue between the first jaw 50 and the second jaw 60 is cut between the cutting edge 53 at the first surface area 52 of the first jaw 50 and the cutting edge 63 at the first surface area 62 of the second jaw 60 when the first jaw 50 is moved into the fenestration 61 in the second jaw 60.

Figure 2 shows a schematic representation of the instrument 10 described above with reference to figure 1 in a second, closed configuration. The configuration of the instrument 10 displayed in figure 2 differs from the configuration displayed in figure 1 in that the first jaw 50 is located inside a fenestration 61 encircled by the second jaw 60. The first jaw 50 essentially or entirely fills the fenestration 61 in the second jaw 60.

In the closed configuration displayed in figure 2, the first and second jaws 50, 60 together form a tool for blunt dissection, in particular for elevation or separation of tissue layers. For this purpose, the first and second jaws 50, 60 are wedge-shaped, viz. their heights (measured in the plane of projection of figures 1 and 2 in a direction orthogonal to the jaws' 50, 60 longitudinal extension) decrease from the body member 40 to the distal end 32 of the tool 30. As the first jaw 50 is located within the fenestration 61 of the second jaw 60, the total height of the closed jaws is small compared to a conventional jawed instrument. When in the configuration shown in figure 2, the cutting edges 66, 52, 53, 63 are all shielded from damaging contact with tissue by the other jaw.

When, in the open configuration of the tool 30 displayed in figure 1, tissue is located between the first jaw 50 and the second jaw 60 and thereafter the first and second jaws 50, 60 approach each other, the tool 30 is transformed towards the closed configuration displayed in figure 2, and the tissue is cut between the cutting edge 53 at the first jaw 50 and the cutting edge 63 at the second jaw 60.

Figure 3 shows another schematic representation of the instrument 10 described above with reference to figures 1 and 2 in a further configuration.

The configuration displayed in figure 3 is similar to the closed configuration displayed in figure 2 in that the first jaw 50 is positioned in the fenestration 61 of the second jaw 60. Thus, the configuration of the tool 30 displayed in figure 3 is a closed configuration like the configuration displayed in figure 2.

The configuration in figure 3 differs from the configuration displayed in figure 2 in that the angular position of both first and second jaws 50, 60 relative to the body member 40 and, thus, relative to the shaft 14 and the longitudinal axis 18 of the shaft 14 is different. While in the closed configuration displayed in figure 2, both first and second jaws 50, 60 are positioned as a straight or linear distal extension of the shaft 14, in the configuration displayed in figure 3, the first and second jaws 50, 60 form an angled or bent distal extension of the shaft 14.

Thus, the tool 30 facilitates blunt dissection of tissue at a range of different angular positions of the first and second jaws 50, 60 relative to the shaft 14. If tissue needs to be elevated at different angles, the surgeon does not need to exchange the instrument but can simply adjust the tool by altering the angular position of the first and second jaws 50, 60 relative to the shaft 14.

Similar to the closed configurations displayed in figures 2 and 3 with different angular positions of the first and second jaws 50, 60 relative to the body member 40 and the shaft 14, an open configuration as displayed in figure 1 can be provided with a range of different angular positions of the first and second jaws 50, 60 relative to the body member 40 and the shaft 14. The adjustability of the angular positions of both first and second jaws 50, 60 provides a flexibility superseding many changes of instruments during a microinvasive surgical operation and considerably reduces the risk of trauma to the patient and the time required for the operation.

Figure 4 shows another schematic representation of the instrument described above with reference to figures 1 through 3 in a further configuration. The configuration displayed in figure 4 is an open configuration similar to the open configuration displayed in figure 1 but differs from the configuration displayed in figure 1 in that the first jaw 50 is located at the opposite side of the second jaw 60. Therefore, the second surface area 55 of the first jaw 50 faces the second surface area 65 of the second jaw 60. The first surface area 52 of the first jaw 50 and the first surface area 62 of the second jaw 60 are averted from each other. If the characteristics of the second surface areas 55, 65 of the jaws 50, 60 are different from the characteristics of the first surface areas 52, 62 of the first and second jaws 50, 60, the open configuration displayed in figure 4 can provide a functionality different from the functionality of the open configuration displayed in figure 1.

In particular, the lateral edge 56 of the second surface area 55 of the first jaw 50 is blunt rather than cutting. In this case, tissue between the first and second jaws 50, 60 is not cut but merely grasped when the first and second jaws 50, 60 are, starting from the open configuration displayed in figure 4, moved towards each other. For this purpose, the cutting edge 66 at the second surface area 65 at the second jaw 60 can be positioned distant from the fenestration 61. As an alternative, the cutting edge 66 can be replaced by a blunt edge or omitted.

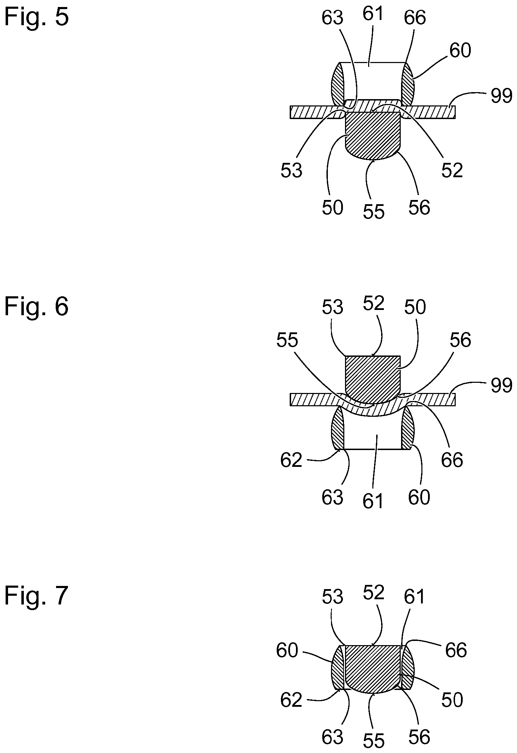

Figure 5 shows a schematic representation of a cross section of the first and second jaws 50, 60 of the tool 30 of the instrument 10 described above with reference to figures 1 through 4. The sectional plane of figure 5 is essentially orthogonal to the longitudinal axes of the first and second jaws 50, 60.

Figure 5 displays the open configuration described above with reference to figure 1. The cutting edges 53, 63 at the first surface areas 52, 62 of the first and second jaws 50, 60 are facing each other. When the first and second jaws 50, 60 are moved towards each other, tissue 99 between the first and second jaws 50, 60 is cut between the cutting edges 53, 63 sliding against each other or passing each other with a sufficiently small distance or clearance. Therefore, the open configuration displayed in figures 1 and 5 is called a cutting open configuration.

Figure 6 shows a further schematic representation of a cross section of the first and second jaws 50, 60 of the tool 30 of the instrument 10 described above with reference to figures 1 through 5. Again, the sectional plane of figure 6 is essentially orthogonal to the longitudinal axes of the first and second jaws 50, 60.

Figure 6 displays a cross section in the open configuration described above with reference to figure 4. Since the edge of the second surface area 55 of the first jaw 50 is blunt and the edge 66 at the second surface area 65 of the second jaw 60 is distant from the fenestration 61, tissue 99 between the first and second jaws 50, 60 is grasped rather than cut when the first and second jaws 50, 60 are moved towards each other. Therefore, the open configuration displayed in figures 4 and 6 is called a grasping open configuration.

Figure 7 shows a further schematic representation of a cross section through the first and second jaws 50, 60 of the tool 30 of the instrument 10 described above with reference to figures 1 through 6. Again, the sectional plane of figure 7 is essentially orthogonal to the longitudinal axes of the first and second jaws 50, 60.

In figure 7, a closed configuration as described above with reference to figures 2 and 3 is displayed. The first jaw 50 is accommodated inside the fenestration 61 encircled by the second jaw 60. Cutting edges 53, 63, 66 at the first and second jaws 50, 60 are less exposed than in the open configurations described above with reference to figures 1, 4, 5 and 6. The proximity of the second jaw 60 to the cutting edge 53 at the first surface area 52 at the first jaw 50 and the proximity of the first jaw 50 to the cutting edge 63 at the first surface area 62 at the second jaw 60 prevent these cutting edges 53, 63 from cutting tissue.

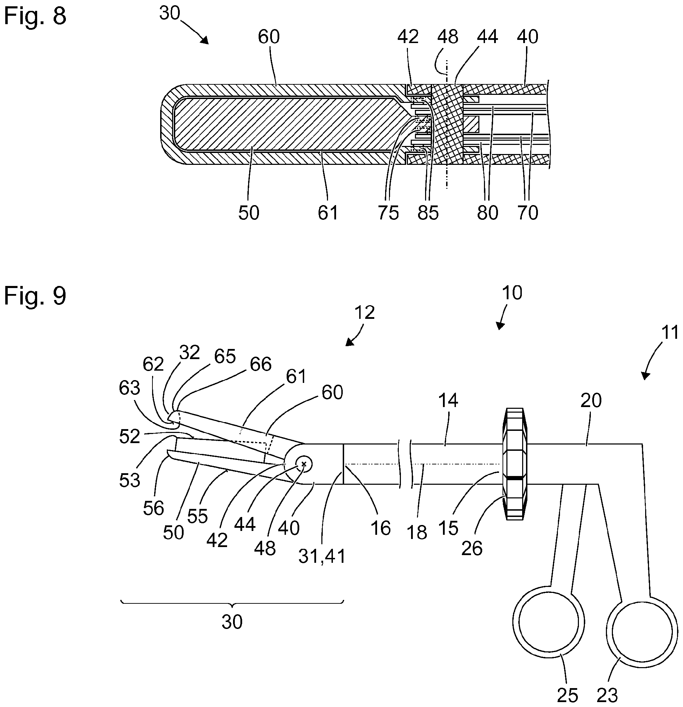

Figure 8 shows a schematic representation of a further cross section through the tool 30 of the instrument described above with reference to figures 1 through 7. The sectional plane of figure 8 is orthogonal to the plane of projection of figures 1 through 4 and comprises the longitudinal axes 18 of the shaft 14 (which itself is not displayed in figure 8).

In figure 8, the U-shape of the second jaw 60, the fenestration 61 encircled by the second jaw 60 and the first jaw 50 located in the fenestration 61 are visible.

In the example displayed in figures 1 through 8, the axis 48 defined by the axle 44 and the longitudinal axis 18 of the shaft are within the same plane which is the sectional plane of figure 8. Pins 75, 85 at connection rods 70, 80 coupling the first and second jaws 50, 60 to respective transmission members in the shaft 14 (not shown in figure 8) and holes in the first and second jaws 50, 60 corresponding to the pins 75, 85 are out of the sectional plane of figure 8 and, thus, are indicated in dashed lines only.

Figure 9 shows a schematic representation of another microinvasive surgical instrument 10. Many features, characteristics and functionalities of the instrument displayed in figure 9 are similar to those of the instrument described above with reference to figures 1 through 8.

The instrument displayed in figure 9 differs from the instrument described above with reference to figures 1 through 8 in particular in the shape of the first and second jaws 50, 60. The second jaw 60 provides the shape of an O entirely encircling a fenestration 61. The proximal and distal ends of the fenestration 61 are indicated by dashed lines.

In the configuration displayed in figure 9, the first jaw 50 is partially accommodated in the fenestration 61. The first jaw 50 cannot be entirely passed through the fenestration 61 and, therefore, always remains on the same side of the second jaw 60.

In all the embodiments and variations described above with reference to figures 1 through 10, each of the first and second surface areas 52, 55, 62, 65 of the first and second jaws 50, 60 can be corrugated for an increased retention or holding force and a reduced risk that tissue to be grasped or cut by the tool slips away. For instance, transverse grooves or notches (parallel to the respective surface area but orthogonal to the longitudinal axis of the respective jaw 50, 60) can be provided. As far as cutting edges 53, 63 confine the surface areas 52, 62, a small distance between the corrugation and the cutting edge 53, 63 can be provided allowing uncorrugated cutting edges 53, 63. A centrally located raised grip feature may be included on the surface area 52 so that when the jaws 50, 60 are closed together they will come into contact with and grasp tissue before the opposing cutting edges 53, 63 close together and cut the tissue. The grip feature would not directly abut the cutting edges 53,63.

### List of reference numerals

- 10: microinvasive surgical instrument
- 11: proximal end of the instrument 10
- 12: distal end of the instrument 10
- 14: shaft of the instrument 10
- 15: proximal end of the shaft 14
- 16: distal end of the shaft 14
- 18: longitudinal axis of the shaft 14 of the instrument 10
- 20: handle assembly of the microinvasive surgical instrument 10, connected to the proximal end 15 of the shaft 14
- 23: fixed handle of the handle assembly 20
- 25: movable handle of the handle assembly 20
- 26: control of the handle assembly 20
- 30: tool at the distal end 12 of the instrument 10
- 31: proximal end of the tool 30, coupled to the distal end 12 of the instrument 10
- 32: distal end of the tool 30
- 40: body member of the tool 30
- 41: proximal end of the body member 40
- 42: distal end of the body member 40
- 44: axle connecting body member 40, first jaw 50 and second jaw 60
- 48: axis defined by axle 44
- 50: first jaw of the tool 30
- 52: first surface area of the first jaw 50, in particular corrugated
- 53: edge, optionally cutting edge, of the first surface ares 52 of the first jaw 50
- 55: second surface area of the first jaw 50, in particular smooth
- 56: edge; optionally cutting edge, of the second surface 55 of the first jaw 50
- 60: second jaw of the tool 30
- 61: fenestration in the second jaw 60
- 62: first surface area of the second jaw 60, in particular corrugated, in particular for grasping, in particular U-shaped or O-shaped
- 63: edge, optionally cutting edge, of the first surface area 63 of the second jaw 60, in particular U-shaped or O-shaped
- 65: second surface area of the second jaw 60, in particular U-shaped or O-shaped
- 66: edge, optionally cutting edge, of the second surface area 65 of the second jaw 60, in particular U-shaped or O-shaped
- 70: first connection rod coupling a transmission member to the first jaw 50
- 75: pin coupling the first connection rod 70 to the first jaw 50
- 80: second connection rod coupling a transmission member to the second jaw 60
- 85: pin coupling the second connection rod 80 to the second jaw 60
- 99: tissue

## Claims

1. **Tool** (30) for a microinvasive surgical instrument (10), comprising:
a **body member** (40), the **proximal end** (41) of the body member (40) being mechanically connected to or being configured to be mechanically coupled to a distal end (16) of a shaft (14) of a microinvasive surgical instrument (10);
a **first jaw** (50) mechanically connected to the body member (40) in an articulated manner;
a **second jaw** (60) mechanically connected to the body member (40) in an articulated manner;
wherein the first jaw (50) and the second jaw (60) are configured to be **pivoted independently** from each other,
wherein the **second jaw** (60) provides the shape of a **'U'** or **'O'** at least partially encircling a fenestration (61),
wherein the tool (30) provides **open configurations** with an angular distance between the first jaw (50) and the second jaw (60),
wherein the tool (30) provides a **closed configuration** with the first jaw (50) at least partially accommodated in or capping the fenestration (61) of the second jaw (60).

2. Tool (30) according to the preceding claim, wherein
the second jaw (60) provides a **cutting edge** (66) exposed and averted from the first jaw (50) in at least one configuration of the tool (30).

3. Tool (30) according to one of the preceding claims, wherein
in the closed configuration, the contour of the first and second jaws (50, 60) is essentially **wedge**-shaped.

4. Tool (30) according to one of the preceding claims, wherein
the cross section of the fenestration (61) at least partially encircled by the second jaw (60) is complementary to the cross section of the first jaw (50) such that the first jaw (50) can be **passed through the fenestration** (61) from a first side of the second jaw (60) to a second side of the second jaw (60) averted from the first side of the second jaw (60).

5. Tool (30) according to claim 4, wherein the first jaw (50) provides, at sides averted from each other, a first surface area (52) with a U-shaped or O-shaped cutting edge (53) and a second surface area (55) with a U-shaped or O-shaped blunt edge (56),
the second jaw (60) provides, at sides averted from each other, a first U-shaped or O-shaped surface area (62) with a U-shaped or O-shaped cutting edge (63) and a second U-shaped or O-shaped surface area (65) with a U-shaped or O-shaped blunt edge (66),
in a **cutting open configuration** of the tool (30), the cutting edge (63) at the second jaw (60) faces the cutting edge (53) at the first jaw (50),
in a **grasping open configuration** of the tool (30), the blunt edge (66) at the second jaw (60) faces the blunt edge (56) at the first jaw (50).

6. Tool (30) according to claim 5, wherein in a **cutting action** transforming the tool (30) from the cutting open configuration towards the closed configuration, the cutting edge (53) at the first jaw (50) slides against or passes the cutting edge (63) at the second jaw (60) in a tissue cutting manner.

7. Tool (30) according to one of claims 5 and 6, wherein
in a **grasping action** transforming the tool (30) from the grasping open configuration to the closed configuration, the blunt edge (56) at the first jaw (50) is moved towards the blunt edge (66) at the second jaw (60).

8. Tool (30) according to one of the preceding claims, wherein both the first jaw (50) and the second jaw (60) are pivotable about the **same pivot axis** (48).

9. Tool (30) according to one of the preceding claims, wherein the tool (30) provides at least two configurations of:
an **elevating closed configuration** in which the first jaw (50) is arranged at least partially inside the fenestration (61) in the second jaw (60), wherein the second jaw (60), by abutting on or being located close to a cutting edge (53) at the first jaw (50), prevents the cutting edge (53) at the first jaw (50) from cutting tissue, and the first jaw (50), by abutting on or being located close to a cutting edge (63) at the second jaw (60), prevents the cutting edge (63) at the second jaw (60) from cutting tissue;
a **grasping open configuration,** tissue being grasped between the first jaw (50) and the second jaw (60) when the first and second jaws (50, 60) are moved towards each other from the grasping open configuration towards the closed configuration;
a **cutting open configuration,** tissue being cut between cutting edges (53, 63) at the first and second jaws (50, 60) sliding against or passing each other when the first and second jaws (50, 60) are moved towards each other from the cutting open configuration towards the closed configuration;
a **curettage configuration** in which a cutting edge (66) at the second jaw (60) averted from the first jaw (50) is exposed and configured to cut tissue when the tool (30) is moved parallel to a surface of the tissue.

10. Tool (30) according to claim 9, wherein in the **elevating closed configuration,** the first and second jaws (50, 60) are in any angular position within a first predetermined range of angular positions relative to the body member (40), with a first predetermined angular relation between the first jaw (50) and the second jaw (60);
in the **grasping open configuration,** each of the first jaw (50) and the second jaw (60) is in any angular position within a respective second predetermined range of angular positions relative to the body member (40), with a second predetermined angular relation between the first jaw (50) and the second jaw (60);
in the **cutting open configuration,** each of the first jaw (50) and the second jaw (60) is in any angular position within a respective third predetermined range of angular positions relative to the body member (40), with a third predetermined angular relation between the first jaw (50) and the second jaw (60);
in the **curettage configuration,** the first and second jaws (50, 60) are in any angular position within a fourth predetermined range of angular positions relative to the body member (40), with a fourth predetermined angular relation between the first jaw (50) and the second jaw (60).

11. **Microinvasive surgical instrument** (10), comprising:
a **shaft** (14) with a proximal end (15) and a distal end (16);
a **tool** (30) according to one of the preceding claims, wherein the proximal end (41) of the body member (40) of the tool (30) is mechanically connected to or is configured to be mechanically coupled to the distal end (16) of the shaft (14);
a **handle assembly** (20) comprising a fixed handle (23) mechanically connected to or configured to be mechanically coupled to the proximal end (15) of the shaft (14) and controls (25, 26) manually movable relative to the fixed handle and mechanically coupled to the first and second jaws (50, 60).

12. Microinvasive surgical instrument (10) according to claim 11, wherein the handle assembly is configured to **fix** at least one of the first jaw (50) and the second jaw (60) **in a manually adjustable position** within a predetermined range of positions relative to the body member (40).

## Patentansprüche

1. Werkzeug (30) für ein mikroinvasives chirurgisches Instrument (10), das Folgendes umfasst:
ein Körperbauteil (40), wobei das proximale Ende (41) des Körperbauteils (40) mit einem distalen Ende (16) eines Schaftes (14) eines mikroinvasiven chirurgischen Instruments (10) mechanisch verbunden ist oder dazu ausgelegt ist, mit diesem mechanisch gekoppelt zu werden;
eine erste Backe (50), die in einer gelenkigen Weise mit dem Körperbauteil (40) mechanisch verbunden ist;
eine zweite Backe (60), die in einer gelenkigen Weise mit dem Körperbauteil (40) mechanisch verbunden ist;
wobei die erste Backe (50) und die zweite Backe (60) dazu ausgelegt sind, unabhängig voneinander geschwenkt zu werden,
wobei die zweite Backe (60) die Form eines "U" oder "O", das zumindest teilweise ein Fenster (61) umschließt, aufweist,
wobei das Werkzeug (30) offene Konfigurationen mit einem Winkelabstand zwischen der ersten Backe (50) und der zweiten Backe (60) aufweist,
wobei das Werkzeug (30) eine geschlossene Konfiguration, bei der die erste Backe (50) zumindest teilweise in dem Fenster (61) der zweiten Backe (60) aufgenommen ist oder dieses abdeckt, aufweist.

2. Werkzeug (30) nach dem vorhergehenden Anspruch, wobei
die zweite Backe (60) eine Schneidkante (66) bereitstellt, die in zumindest einer Konfiguration des Werkzeugs (30) freiliegt und von der ersten Backe (50) abgewandt ist.

3. Werkzeug (30) nach einem der vorhergehenden Ansprüche, wobei
in der geschlossenen Konfiguration die Kontur der ersten und der zweiten Backe (50, 60) im Wesentlichen keilförmig ist.

4. Werkzeug (30) nach einem der vorhergehenden Ansprüche, wobei
der Querschnitt des Fensters (61), das zumindest teilweise durch die zweite Backe (60) umschlossen ist, komplementär zu dem Querschnitt der ersten Backe (50) ist, sodass die erste Backe (50) von einer ersten Seite der zweiten Backe (60) zu einer zweiten Seite der zweiten Backe (60), die von der ersten Seite der zweiten Backe (60) abgewandt ist, durch das Fenster (61) geführt werden kann.

5. Werkzeug (30) nach Anspruch 4, wobei
die erste Backe (50) an Seiten, die voneinander abgewandt sind, einen ersten Oberflächenbereich (52) mit einer U-förmigen oder O-förmigen Schneidkante (53) und einen zweiten Oberflächenbereich (55) mit einer U-förmigen oder O-förmigen stumpfen Kante (56) aufweist,
die zweite Backe (60) an Seiten, die voneinander abgewandt sind, einen ersten U-förmigen oder O-förmigen Oberflächenbereich (62) mit einer U-förmigen oder O-förmigen Schneidkante (63) und einen zweiten U-förmigen oder O-förmigen Oberflächenbereich (65) mit einer U-förmigen oder O-förmigen stumpfen Kante (66) aufweist,
in einer offenen Schneidkonfiguration des Werkzeugs (30) die Schneidkante (63) an der zweiten Backe (60) der Schneidkante (53) an der ersten Backe (50) gegenüberliegt,
in einer offenen Greifkonfiguration des Werkzeugs (30) die stumpfe Kante (66) an der zweiten Backe (60) der stumpfen Kante (56) an der ersten Backe (50) gegenüberliegt.

6. Werkzeug (30) nach Anspruch 5, wobei
bei einer Schneidaktion, bei der das Werkzeug (30) von der offenen Schneidkonfiguration in die geschlossene Konfiguration übergeht, die Schneidkante (53) an der ersten Backe (50) in einer Gewebe schneidenden Weise an der Schneidkante (63) an der zweiten Backe (60) entlang gleitet oder diese passiert.

7. Werkzeug (30) nach einem der Ansprüche 5 und 6, wobei:
bei einer Greifaktion, bei der das Werkzeug (30) von der offenen Greifkonfiguration in die geschlossene Konfiguration übergeht, die stumpfe Kante (56) an der ersten Backe (50) zu der stumpfen Kante (66) an der zweiten Backe (60) bewegt wird.

8. Werkzeug (30) nach einem der vorhergehenden Ansprüche, wobei
sowohl die erste Backe (50) als auch die zweite Backe (60) um die gleiche Schwenkachse (48) schwenkbar sind.

9. Werkzeug (30) nach einem der vorhergehenden Ansprüche, wobei das Werkzeug (30) mindestens zwei der folgenden Konfigurationen aufweist:
eine geschlossene anhebende Konfiguration, bei der die erste Backe (50) zumindest teilweise innerhalb des Fensters (61) in der zweiten Backe (60) angeordnet ist, wobei die zweite Backe (60) dadurch, dass sie an einer Schneidkante (53) an der ersten Backe (50) anliegt oder nahe derselben angeordnet ist, verhindert, dass die Schneidkante (53) an der ersten Backe (50) Gewebe schneidet, und wobei die erste Backe (50) dadurch, dass sie an einer Schneidkante (63) an der zweiten Backe (60) anliegt oder nahe derselben befindlich ist, verhindert, dass die Schneidkante (63) an der zweiten Backe (60) Gewebe schneidet;
eine offene Greifkonfiguration, wobei das Gewebe zwischen der ersten Backe (50) und der zweiten Backe (60) gegriffen wird, wenn die erste und die zweite Backe (50, 60) von der offenen Greifkonfiguration in Richtung der geschlossenen Konfiguration aufeinander zu bewegt werden;
eine offene Schneidkonfiguration, wobei das Gewebe zwischen Schneidkanten (53, 63) an der ersten und der zweiten Backe (50, 60) geschnitten wird, die aneinander entlang gleiten oder einander passieren, wenn die erste und die zweite Backe (50, 60) von der offenen Schneidkonfiguration in Richtung der geschlossenen Konfiguration aufeinander zu-bewegt werden;
eine Kürettagekonfiguration, bei der eine Schneidkante (66) an der zweiten Backe (60), die von der ersten Backe (50) abgewandt ist, freiliegt und ausgelegt ist zum Schneiden von Gewebe, wenn das Werkzeug (30) parallel zu einer Oberfläche des Gewebes bewegt wird.

10. Werkzeug (30) nach Anspruch 9, wobei
in der geschlossenen anhebenden Konfiguration die erste und die zweite Backe (50, 60) in einer beliebigen Winkelposition innerhalb eines ersten vorbestimmten Bereichs von Winkelpositionen relativ zu dem Körperbauteil (40) sind, mit einer ersten vorbestimmten Winkelbeziehung zwischen der ersten Backe (50) und der zweiten Backe (60);
in der offenen Greifkonfiguration sowohl die erste Backe (50) als auch die zweite Backe (60) in einer beliebigen Winkelposition innerhalb eines entsprechenden zweiten vorbestimmten Bereichs von Winkelpositionen relativ zu dem Körperbauteil (40) sind, mit einer zweiten vorbestimmten Winkelbeziehung zwischen der ersten Backe (50) und der zweiten Backe (60);
in der offenen Schneidkonfiguration sowohl die erste Backe (50) als auch die zweite Backe (60) in einer beliebigen Winkelposition innerhalb eines entsprechenden dritten vorbestimmten Bereichs von Winkelpositionen relativ zu dem Körperbauteil (40) sind, mit einer dritten vorbestimmten Winkelbeziehung zwischen der ersten Backe (50) und der zweiten Backe (60);
in der Kürettagekonfiguration die erste und die zweite Backe (50, 60) in einer beliebigen Winkelposition innerhalb eines vierten vorbestimmten Bereichs von Winkelpositionen relativ zu dem Körperbauteil (40) sind, mit einer vierten vorbestimmten Winkelbeziehung zwischen der ersten Backe (50) und der zweiten Backe (60).

11. Mikroinvasives chirurgisches Instrument (10), das Folgendes umfasst:
einen Schaft (14) mit einem proximalen Ende (15) und einem distalen Ende (16);
ein Werkzeug (30) nach einem der vorhergehenden Ansprüche, wobei das proximale Ende (41) des Körperbauteils (40) des Werkzeugs (30) mit einem distalen Ende (16) des Schaftes (14) mechanisch verbunden ist oder dazu ausgelegt ist, mit diesem mechanisch gekoppelt zu werden;
eine Griffanordnung (20), umfassend einen festen Griff (23), der mit dem proximalen Ende (15) des Schaftes (14) mechanisch verbunden ist oder dazu ausgelegt ist, mit diesem mechanisch gekoppelt zu werden, und Steuereinrichtungen (25, 26), die manuell relativ zu dem festen Griff bewegbar sind und mit der ersten und der zweiten Backe (50, 60) mechanisch gekoppelt sind.

12. Mikroinvasives chirurgisches Instrument (10) nach Anspruch 11, wobei
die Griffanordnung ausgelegt ist zum Fixieren der ersten Backe (50) und/oder der zweiten Backe (60) in einer manuell einstellbaren Position innerhalb eines vorbestimmten Bereichs von Positionen relativ zu dem Körperbauteil (40).

## Revendications

1. Outil (30) pour un instrument chirurgical micro-invasif (10), comprenant :
un élément formant un corps (40), l'extrémité proximale (41) de l'élément corps (40) étant reliée mécaniquement ou étant conçue pour être couplée mécaniquement à une extrémité distale (16) d'un axe (14) d'un instrument chirurgical micro-invasif (10) ;
une première pince (50) reliée mécaniquement à l'élément corps (40) de manière articulée ;
une seconde pince (60) reliée mécaniquement à l'élément corps (40) de manière articulée ;
dans lequel la première pince (50) et la seconde pince (60) sont conçues pour être pivotées indépendamment l'une de l'autre,
dans lequel la seconde pince (60) présente la forme d'un U ou d'un O entourant au moins en partie une fenestration (61),
l'outil (30) permettant des configurations ouvertes avec une distance angulaire entre la première pince (50) et la seconde pince (60),
l'outil (30) permettant une configuration fermée avec la première pince (50) au moins en partie logée dans ou recouvrant la fenestration (61) de la seconde pince (60).

2. Outil (30) selon la revendication précédente, dans lequel
la seconde pince (60) présente un bord de coupe (66) exposé et opposé à la première pince (50) dans au moins une configuration de l'outil (30).

3. Outil (30) selon l'une des revendications précédentes, dans lequel
dans la configuration fermée, le contour des première et seconde pinces (50, 60) est essentiellement en forme de coin.

4. Outil (30) selon l'une des revendications précédentes, dans lequel
la section transversale de la fenestration (61) au moins en partie entourée par la seconde pince (60) est complémentaire à la section transversale de la première pince (50) afin que la première pince (50) puisse passer à travers la fenestration (61) d'un premier côté de la seconde pince (60) à un second côté de la seconde pince (60) opposé au premier côté de la seconde pince (60).

5. Outil (30) selon la revendication 4, dans lequel
la première pince (50) présente, sur des côtés opposés l'un à l'autre, une première surface (52) dotée d'un bord de coupe en forme de U ou en forme de O (53) et une seconde surface (55) dotée d'un bord émoussé en forme de U ou en forme de O (56),
la seconde pince (60) présente, sur des côtés opposés l'un à l'autre, une première surface en forme de U ou en forme de O (62) dotée d'un bord de coupe en forme de U ou en forme de O (63) et une seconde surface en forme de U ou en forme de O (65) dotée d'un bord émoussé en forme de U ou en forme de O (66),
dans une configuration ouverte de coupe de l'outil (30), le bord de coupe (63) de la seconde pince (60) fait face au bord de coupe (53) de la première pince (50),
dans une configuration ouverte de saisie de l'outil (30), le bord émoussé (66) de la seconde pince (60) fait face au bord émoussé (56) de la première pince (50).

6. Outil (30) selon la revendication 5, dans lequel
lors d'une action de coupe transformant l'outil (30) de la configuration ouverte de coupe vers la configuration fermée, le bord de coupe (53) de la première pince (50) coulisse contre ou dépasse le bord de coupe (63) de la seconde pince (60) de manière à découper des tissus.

7. Outil (30) selon l'une des revendications 5 et 6, dans lequel
lors d'une action de saisie transformant l'outil (30) de la configuration ouverte de saisie vers la configuration fermée, le bord émoussé (56) de la première pince (50) est déplacé vers le bord émoussé (66) de la seconde pince (60).

8. Outil (30) selon l'une des revendications précédentes, dans lequel la première pince (50) et la seconde pince (60) peuvent toutes deux être pivotées autour du même axe de pivot (48).

9. Outil (30) selon l'une des revendications précédentes, l'outil (30) présentant au moins deux des configurations suivantes :
une configuration fermée d'élévation dans laquelle la première pince (50) est disposée au moins en partie à l'intérieur de la fenestration (61) dans la seconde pince (60), dans lequel la seconde pince (60), en venant en butée contre ou en étant située près d'un bord de coupe (53) de la première pince (50), empêche le bord de coupe (53) de la première pince (50) de découper des tissus, et la première pince (50), en venant en butée contre ou en étant située près d'un bord de coupe (63) de la seconde pince (60), empêche le bord de coupe (63) de la seconde pince (60) de découper des tissus ;
une configuration ouverte de saisie, les tissus étant saisis entre la première pince (50) et la seconde pince (60) lorsque les première et seconde pinces (50, 60) sont déplacées l'une vers l'autre de la configuration ouverte de saisie vers la configuration fermée ;
une configuration ouverte de coupe, les tissus étant découpés entre les bords de coupe (53, 63) des première et seconde pinces (50, 60) coulissant l'une contre l'autre ou se dépassant l'une l'autre lorsque les première et seconde pinces (50, 60) sont déplacées l'une vers l'autre de la configuration ouverte de coupe vers la configuration fermée ;
une configuration de curetage dans laquelle un bord de coupe (66) de la seconde pince (60) opposé à la première pince (50) est exposé et configuré pour découper des tissus lorsque l'outil (30) est déplacé parallèlement à la surface des tissus.

10. Outil (30) selon la revendication 9, dans lequel
dans la configuration fermée d'élévation, les première et seconde pinces (50, 60) se trouvent dans toute position angulaire à l'intérieur d'une première plage prédéterminée de positions angulaires par rapport à l'élément corps (40), avec une première relation angulaire prédéterminée entre la première pince (50) et la seconde pince (60) ;
dans la configuration ouverte de saisie, chacune de la première pince (50) et de la seconde pince (60) se trouve dans toute position angulaire à l'intérieur d'une deuxième plage prédéterminée respective de positions angulaires par rapport à l'élément corps (40), avec une deuxième relation angulaire prédéterminée entre la première pince (50) et la seconde pince (60) ;
dans la configuration ouverte de coupe, chacune de la première pince (50) et de la seconde pince (60) se trouve dans toute position angulaire à l'intérieur d'une troisième plage prédéterminée respective de positions angulaires par rapport à l'élément corps (40), avec une troisième relation angulaire prédéterminée entre la première pince (50) et la seconde pince (60) ;
dans la configuration de curetage, les première et seconde pinces (50, 60) se trouvent dans toute position angulaire à l'intérieur d'une quatrième plage prédéterminée de positions angulaires par rapport à l'élément corps (40), avec une quatrième relation angulaire prédéterminée entre la première pince (50) et la seconde pince (60).

11. Instrument chirurgical micro-invasif (10), comprenant :
un axe (14) possédant une extrémité proximale (15) et une extrémité distale (16) ;
un outil (30) selon l'une des revendications précédentes, dans lequel l'extrémité proximale (41) de l'élément corps (40) de l'outil (30) est reliée mécaniquement ou est conçue pour être couplée mécaniquement à l'extrémité distale (16) de l'axe (14) ;
un ensemble poignée (20) comprenant une poignée fixe (23) reliée mécaniquement à ou conçu pour être couplé mécaniquement à l'extrémité proximale (15) de l'axe (14) et des commandes (25, 26) pouvant être déplacées manuellement par rapport à la poignée fixe et couplées mécaniquement aux première et seconde pinces (50, 60).

12. Instrument chirurgical micro-invasif (10) selon la revendication 11, dans lequel
l'ensemble poignée est conçu pour fixer au moins l'une de la première pince (50) et de la seconde pince (60) dans une position pouvant être réglée manuellement à l'intérieur d'une plage prédéterminée de positions par rapport à l'élément corps (40).
